# EUROPEAN PATENT APPLICATION

(11) **EP 1 695 976 A1**
(43) Date of publication of application: **30.08.2006**
(21) Application number: 04797343.3
(22) Date of filing: 18.11.2004
(51) Int. Cl.: C07D 487/04, A61P 15/10

(54) **PYRAZOLOPYRIMIDINETHIONE DERIVATIVES, SALTS AND SOLVATES THEREOF, PREPARATION METHODS AND USE THEREOF**

(30) Priority: 18.12.2003 CN 200310118481
(71) Applicant: Institute of Radiation Medicine Academy of Military Medical Sciences of the Pla, Beijing 100850 (CN)
(72) Inventor: LI, Shuxin, Beijing 100850 (CN); REN, Jianping, Beijing 100850 (CN); ZHAO, Yanjin, Beijing 100850 (CN); LV, Qiujun, Beijing 100850 (CN); GUO, Jinhua, Beijing 100850 (CN)
(74) Representative: Crooks, Elizabeth Caroline
(86) International application number: PCT/CN2004/001312
(87) International publication number: WO 2005/058899

(57) **Abstract**

The present invention disclosed the pyrazolopyrimidinethione derivatives, salts and solvates thereof, preparation methods and use thereof. The pyrazolopyrimidinethione derivatives according to the present invention possess the structure of general formula I , wherein, R₁, R₂, R₃, and R₄ represent alkyl, alkenyl, or aryl; R₅ represents hydrogen, alkyl, alkenyl, alkoxy, cycloalkyloxy, aryl, or alkylacyl; and R₆ represents hydrogen, alkyl, alkenyl, cycloalkyloxy, or alkylacyl. The pharmaceuticals containing the compound of the present invention and used for the treatment of impotence and sexlessness have the advantages of high selectivity over PDEV, long action time, and less side reactions, and the pharmaceuticals will arouse no side reactions of blood pressure decreasing and heart rate increasing, and it has broad market propect.

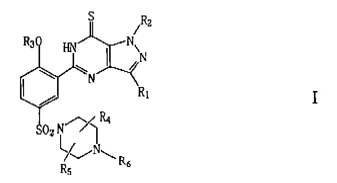

## Description

### FIELD OF THE INVENTION

The invention relates to a high selective phosphodiesterase V inhibitor, pyrazolopyrimidinethione derivatives, and salts and solvates thereof, for preventing and/or treating impotence and frigidity, and their preparation methods and medical applications.

### BACKGROUND OF THE INVENTION

Impotence can be defined as the powerlessness in the male to copulate, including the incapability of penile erection or ejaculation, or both. The prevalence increases with age. It is reported that impotence rate is between 2 and 7% in men younger than 50 years old, and between 18-75% in men 55 and 80 years old. For example, in the USA, it has been estimated that there are up to 10 million impotent males, with the majority suffering from the problems of physiological rather than of psychogenic origin.

Erection of penis relates to relaxation of smooth muscle in the corpus cavernosum penis. Nitrogen oxide is released from the nerve ending and endothelial cells of the corpus cavernosum penis during the sexual stimulation, activating guanyl cyclase and resulting in the increased synthesis of cyclic guanosine monophosphate (cGMP) in the corpus cavernosum smooth muscle cells. cGMP causes the smooth muscle to relax and increases blood flow into the penis, leading to penis erection. The tissue concentration of cGMP is regulated by phosphodiesterases (PDEs). The most plentiful phosphodiesterase in the corpus cavemasum of the penis is cGMP-specific phosphodiesterase V (PDE V). The inhibition of PDE V can increase the level of cGMP and enhance erection function.

The existing pharmaceuticals for treating impotence are phenolamine, ketamine, and prostaglandin E1, some of which belong to those with nerve excitability, having the problems of pharmaceutical addiction and etc.

Sildenafil is a selective phosphodiesterase inhibitor. This compound, its preparation method, and the use of the same for treating cardiovascular disease are disclosed in the invention parent application publication CN1057464A; the use of the compound for preparing the pharmaceuticals of treating the erectile dysfunction of male animals is disclosed in CN 1124926A; and a novel method for preparing sildenafil is disclosed in CN1168376A. Although sildenafil has good potency for the treatment of male erectile dysfunction, it has rather clear side effects with a large dose (50 mg/time or 100 mg/time), such as headache, dizzy, blue-sighted, blood pressure reduction, heart rate increase, and even severe cardiovascular negative effects.

CN 1393444A disclosed a class of compounds:
5-[2-ethoxy-5-(cis-3,5-dimethylpiperazin-1-sulfonyl)phenyl]-1-methyl-3-n-propyl-1,6-dihydro-7H-pyrazolo[4,3d]pyrimidin-7-one compound, which has a higher activity (30mg/d) and lower toxicity than sildenafil, with the LD₅₀ of 772.5-1052.1mg/kg. Chinese Invention Patent (Application No.: 2003101016916) disclosed a group of compounds: 5-[2-ethoxy-5-(cis-3,5-dimethylpiperazine-1-sulfonyl)phenyl]-1-ehtyl-3-n-propyl-1,6-dihydro-7H-pyrazolopyrimidin-7-one, which also has a higher activity than sildenafil. An experiment was conducted with healthy mice, with half male and half female. After the oral administration of said pharmaceutical at the doses of 30mg/kg and 15mg/kg to the mice, as well as sildenafil at the dose of 50mg/kg as a control, within two hours, the times of back climbing on the female mice performed by the male mice were 53, 43, and 19, respectively. The occurrences of back climbing were 62.5%, 56.6%, and 50%. There was a significant difference (P<0.01) compared with the control group with the back climbing of 3 times and the occurrence of 12.6%. The preliminary acute toxicity test shows that the LD₅₀ of said pharmaceutical is greater than 2000mg/kg, which is dramatically higher than that of Sildenafil (LD50 is 635mg/kg). The efficacy on dog shows that said pharmaceutical can dramatically decrease diastolic pressure and increase heart rate, suggesting that the clinical use of said pharmaceutical would have side effects including causing blood pressure decreased and heart rate increased. The side effects were associated with phosphodiesterase isoenzymes, mainly with the low selectivity of phosphodiesterase VI in the eyes and phosphodiesterases I and III in the heart.

### DETAILED DISCRIPTION OF THE INVENTION

One objective of the invention is to provide novel pyrazolopyrimidinethione derivatives, salts and solvates thereof.

Pymzolopyrimidinethione derivatives provided in the invention have the structure of formula I:

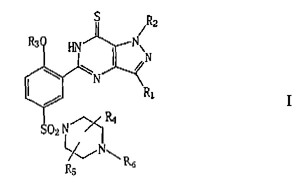

Wherein: R₁, R₂, and R₃ are same or different, and independently are alkyl having 1-6 carbon atoms, alkyl having 1-6 carbon atoms in which at least one hydrogen atom is substituted by alkoxy having 1-6 carbon atoms or cycloalkyloxy having 3-6 carbon atoms, alkenyl having 2-6 carbon atoms, or aryl having 6-10 carbon atoms;

R₄ is alkyl having 1-6 carbon atoms, alkenyl having 2-6 carbon atoms, alkoxy having 1-6 carbon atoms, cycloalkyloxy having 3-6 carbon atoms, aryl having 6-10 carbon atoms, or alkyloyl having 1-6 carbon atoms;

R₅ is hydrogen, alkyl having 1-6 carbon atoms, alkenyl having 2-6 carbon atoms, alkoxy having 1-6 carbon atoms, cycloalkyloxy having 3-6 carbon atoms, aryl having 6-10 carbon atoms, or alkyloyl having 1-6 carbon atoms;

R₆ is hydrogen, alkyl having 1-6 carbon atoms, alkenyl having 3-6 carbon atoms, cycloalkyl having 3-8 carbon atoms, or alkyloyl having 1-6 carbon atoms.

The preferred compounds of the invention have the structure of formula II, wherein: R₁, R₂, R₃, R₄, and R₅ are same or different, and are alkyl having 1-6 carbon atoms, such as methyl, ethyl, propyl, butyl and the like.

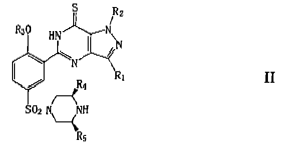

More particularly, the compounds of the present invention include:
5-[2-methoxy-5-(cis-3,5-dimethylpiperazin-1-sulfonyl)phenyl]-1-methyl-3-n-propyl-1,6-dihydro-7H-pyrazolo[4,3-d]pyrimidin-7-thiane;
5-[2-ethoxy-5-(cis-3,5- dimethylpiperazin -1-sulfonyl)phenyl]-1-methyl-3-n-propyl-1,6-dihydro-7H -pyrazolo[4,3-d]pyrimidin-7-thione;
5-[2-propoxy-5-(cis-3,5-dimethylpiperazin-1-sulfonyl)phenyl]-1-methyl-3-n-propyl-1,6-dihydro-7H-pyrazolo[4,3-d]pyrimidin-7-thione;
5-[2-methoxy-5-(cis-3,5-dimethylpiperazin-1-sulfonyl)phenyl]-1-ethyl-3-n-propyl-1,6-dihydro-7H-pyrazolo[4,3-d]pyrimidin-7-thione;
5-[2-ethoxy-5-(cis-3,5- dimethylpiperazin-1-sulfonyl)phenyl]-1-ethyl-3-n-propyl-1,6-dihydro-7H-pyrazolo[4,3-d]pyrimidin-7-thione;or
5-[2-propoxy-5-(cis-3,5-dimethylpiperazine-1-sulfonyl)phenyl]-1-ethyl-3-n-propyl-1,6-dihydro-7H-pyrazolo[4,3-d]pyrimidin-7-thione and so on.

Compounds of the invention contain basic center, which can form salts with acid. The pharmaceutically acceptable salts are preferably nontoxic acid addition salts, for example, methanesulphonate, trifluoromethanesulphonate, formed by a reaction with lower-alkyl-substituted sulfonic acid such as methane sulfonic acid, trifluoromethanesulfonic acid and etc.; p-toluenesulfonate, benzene sulfonate, formed by a reaction with aryl sulfonic acid such as benzene sulfonic acid or p-toluene sulfonic acid; the corresponding salts formed with an organic carboxylic acid, such as acetic acid, fumaric acid, tartaric acid, oxalic acid, maleic acid, malic acid, succinic acid, or citric acid or the like; glucamate or aspartate formed with an amino acid, such as glutamic acid or aspartic acid. Compounds of the invention can form the corresponding salts through a reaction with an inorganic acid, such as hydrohalic acid (e-g., hydrofluoric acid, hydrobromic acid, hydroiodic acid, and hydrochloric acid), nitric acid, sulfuric acid or phosphoric acid or the like. Compound of formula I also provides the pharmaceutically acceptable metal salts, particularly alkali metal salts, such as sodium salts or potassium salts.

Pyrazolopyrimidinethione derivatives of the invention or the solvates of their salts are within the protection scope of the invention. The solvents therefor preferably are water, ethanol, or methanol.

The second objective of the invention is to provide a first method for preparing the pyrazolopyrimidinethione derivatives of the invention.

The first preparation method provided in the invention is to react the compound of formula III with the compound of formula N in a solvent to obtain the compound of formula I.

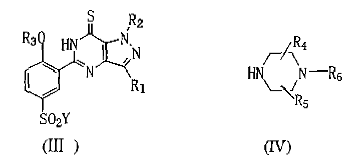

Wherein: R₁, R₂, and R₃ are same or different, and are alkyl having 1-6 carbon atoms, alkyl having 1-6 carbon atoms in which at least one hydrogen atom is substituted by alkoxy having 1-6 carbon atoms or cycloalkyloxy having 3-6 carbon atoms, alkenyl having 2-6 carbon atoms, or aryl having 6-10 carbon atoms;

R₄ is alkyl having 1-6 carbon atoms, alkenyl having 2-6 carbon atoms, alkoxy having 1-6 carbon atoms, cycloalkyloxy having 3-6 carbon atoms, aryl having 6-10 carbon atoms, or alkyloyl having 1-6 carbon atoms;

R₅ is hydrogen, alkyl having 1-6 carbon atoms, alkenyl having 2-6 carbon atoms, aryl having 6-10 carbon atoms, or alkyloyl having 1-6 carbon atoms;

R₆ is hydrogen, alkyl having 1-6 carbon atoms, alkenyl having 3-6 carbon atoms, cycloalkyl having 3-8 carbon atoms, or alkyloyl having 1-6 carbon atoms; and Y is Cl, F, Br, or I.

The reaction is generally conducted at room temperature, and may optionally use a solvent, such as organic solvents including chloroform, dichloromethane, tetrahydrofuran, dioxane, 1,2-dimethoxyethane, ethanol, xylene, toluene, dimethyl sulfoxide, triethylamine and so on.

The salts of the pyrazolopyrimidinethione derivatives of the invention can be obtained by reacting the pyrazolopyrimidinethione derivatives with the pharmaceutically acceptable acids.

The invention further provides another method for preparing the compound of formula I which comprise reacting the compound of formula V with the compound of formula N in a solvent to give the compound of formula VI, and then producing the product by sulfuration of the compound of formula VI.

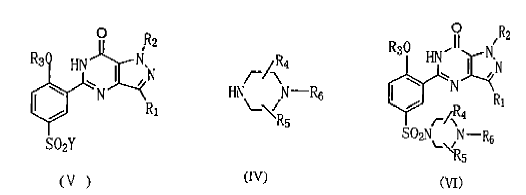

Wherein: R₁, R₂, R₃, R₄, R₅, and R₆ in the compounds of formula IV, V, and VI are defined as those defined in the compound of formula I, and Y is Cl. F, Br, and I.

When compound VI is subjected to sulfuration, the employable sulfurating reagents are phosphorus pentasulfide or 2,4-bis(p-methoxyphenyl)-1,3-dithia-2,4-diphosphetane-2,4-disulfide (Lawesson reagent), and derivatives thereof and the like. The sulfuration of compound VI is conducted in an appropriate solvent such as organic solvents including chloroform, dichloromethane, tetrahydrofuran, dioxane, 1,2-dimethoxyethane, ethanol, xylene, toluene, dimethyl sulfoxide, triethylamine. The reaction temperature is at a temperature in the range of -20° to 200°C.

The starting materials, compound III and V, as used, are synthesized according to the following route, referring to the method disclosed in EP463752:

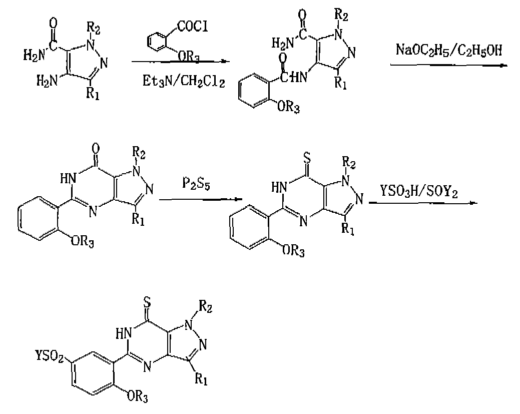

Wherein the definitions for R₁, R₂, and R₃ are the same as those for the compound of formula I , and Y is Cl.

Compound IV may be commercially available or synthetically obtained by conventional methods.

The third objective of the invention is to provide a pharmaceutical which utilizes the invented pyrazolopyrimidinethione derivatives, salts and solvates thereof as the active ingredient for preventing or treating impotence and sex frigidity.

The inventors of the invention confirmed by the experiments that the compounds of the invention can shorten the latent period of the penile erection in rats with testis removed and lengthen the penile swelling period. The compounds of the invention increases the times of back climbing on female mice by male mice. The compounds of the invention have the advantages of small cardiovascular side effects, and will not cause a decrease of the blood pressure and an increase of the heart rate. Each compound of the invention, as well as salts and solvates thereof, all acts as the active ingredient of the pharmaceutical for preventing and/or treating impotence and frigidity (female).

One or more pharmaceutically acceptable carriers can be further contained in the above pharmaceutical. Said carriers include conventional diluter, excipient, filling agent, adhesive, wetting agent, disintegrating agent, surfactant, adsorption carrier, lubricant etc. known in the field of medicine. If necessary, flavoring agent, sweetener and etc. may be added. The pharmaceutical of the invention may be formulated into various forms of tablet, powder, granule, capsule, oral liquid and injectable preparation. Said pharmaceutical in various dosage forms can be prepared according to the conventional methods in the field of medicine.

### PREFERRED EMBODIMENT OF THE INVENTION

Example 1. Synthesis of
5-[2-ethoxy-5-(cis-3,5-dimethylpiperazin-1-sulfonyl)phenyl]-1-ethyl-3-n-propyl-1,6-dihydro-7H-pyraz olo[4,3-d]pyrimidin-7-thione
(In formula II, wherein R₁ is n-propyl, R₂ is ethyl, R₃ is ethyl, and R₄ and R₅ are methyl)
(COMPOUND (1))

Step 1. Synthesis of
4-(2-ethoxybenzamido)-1-ethyl-3-n-propylpyrazol-5-formamidc

10g of 1-ethyl-3-n-propyl-4-aminopyrazole-5-formamide was dissolved in 250ml of dry dichloromethane and 10ml of triethylamine was added in a 500-ml three-necked round-bottomed flask under the protection of N₂, and cooled to below 5°C in an ice-hath. To this solution, 11.3g of o-ethoxybenzoyl chloride was added dropwise and the addition rate was controlled, allowing the internal temperature not to reach a temperature of higher than 5°C. After the addition, the reactants were stirred for another 3h to allow the reaction to happen. The reaction mixture was placed at room temperature ovemight. Upon the completion of the reaction, the reaction mixture was washed with water, aqueous sodium carbonate, and then water. Following this, the reaction mixture was dried and concentrated. The reaction mixture was re-crystallized with n-propyl alcohol to give 13.0g of the title compound Yield: 74.0%.

Melting point (m.p.) of the product is 136-138°C; MS(FAB) 345(M+1).

Step 2. Synthesis of 5-(2-ethoxyphenyl)-1-ethyl-3-n-propyl-1.6-dihydro-7H-pyrazolo[4,3-d]pyrimidin-7-one

1.0g of sodium was added to 75ml of absolute ethanol. After sodium was completely decomposed, 8.0g of the product of step (1) was added and refluxed for 4-5h. Upon the completion of the reaction, the solvent was removed by concentration. After cooling, concentrated hydrochloric acid was added to make the solid separated. The solid was filtered, washed with water, and dried in air. The solid was re-crystallized with methanol to give 6.5 g of the title compound. Yield: 85.7%,

Melting point of the product: m.p.114-15°C; MS (FAB) 327(M+1);

¹HNMR(DMSO-d₆) (ppm): δ0.94(t,3H), δ1.34(t,3H), δ1.40(t,3H), δ1.75(m,2H), δ2.79(t,2H), δ4.14(q,2H), δ4.53(q,2H), δ7.06(t,1H), δ7.15(d,1H), δ7.46(m,1H), δ7.68(m,1H).

Step 3. Synthesis of 5-(2-ethoxyphenyl)-1-ethyl-3-n-propyl-1,6-dihydro-7H-pyrazolo[4,3-d]pyrimidin-7-thione

In a 100-ml three-necked round-bottomed flask, 3.5g of the product from step (2) was dissolved in 30ml of toluene. To the solution, 1.0g of phosphorus pentasulfide were added, and then refluxed for 2 hours. Upon the completion of the reaction, toluene was removed under reduced pressure. The reaction mixture was cooled to room temperature, and dissolved by adding chloroform. The solution was dissolved and washed with 2N NaOH and then washed with water. The organic layer was dried over Na₂SO₄, concentrated, and re-crystallized with methanol. It gave 3.1g of yellow solid. Yield: 84.4%.

The melting point of the product: m.p. 130-131°C;

¹HNMR(DMSO-d₆) (ppm): δ0.94(t,3H), δ1.39(t,3H), δ1.40(t,3H), δ1.77(q,2H), δ2.84(t,2H), δ4.19(q,2H), δ4.95(q,2H), δ7.11(t,1H), δ7.20(d,1H), δ7.53(t,1H), δ7.83(d,1H), δ13.31(s,1H);

MS(FAB) 343 (M+1).

Step 4. Synthesis of 5-(5-chlorosulfonyl-2-ethoxyphenyl)-1-ethyl-3-n-propyl-1,6-dihydro-7H-pyrazolo[4,3-d] pyrimidin-7-thione

5ml of chlorosulfonic acid was added in a 100-ml three-necked round-bottomed flask with electromagnetic stirring. After cooling to 10°C in an ice-bath, 3.0g of the product of step (3) was added in batches. After the completion of the addition, the reactant was reacted for 7 hours at room temperature, and then 5.0ml of thionyl chloride was added. As the reaction mixtures were dropped into the crushed ice, a yellow solid was immediately produced. The yellow solid was extracted with chloroform, washed with water, dried over MgSO₄, and concentrated, to give 3.3g of the title compound. Yield: 84.5%.

Step 5. Synthesis of 5-[2-ethoxy-5-(cis-3,5-dimethylpiperszin-1-sulfonyl)phenyl]-1-ethyl-3-n-propyl-1,6-dihydro-7H-pyrazolo[4,3-d]pyrimidin-7-thione (compound (1))

1.2g of cis-2,6-dimethylpiperazine was dissolved in 5ml of chloroform, to which 3.0g of the product of step (4) was added with cooling in an ice-bath. Upon the completion of the reaction, it was washed with aqueous Na₂CO₃, then with water, dried over Na₂SO₄, and re-crystallized with ethyl acetate, to give 3.2g of a yellow solid. Yield: 81.2%.

The melting point ofthe product: m.p.202-203°C; MS(FAB) 519.2(M+1);

¹HNMR(DMSO-d₆) (ppm): δ0.92-0.96(m,9H), δ1.34-1.40 (m,6H), δ1.72-1.78(m,4H), δ2.75-2.86(m,4H), δ3.47(d,2H), δ4.23(q,2H), δ4.96(q,2H), δ7.39(d,1H), δ7.89(q,1H), δ7.94(d,1H).

The resultant product was confirmed as the desired product.

Example 2. Synthesis of 5-[2-ethoxy-5-(cis-3,5-dimethylpiperazin-1-sulfonyl)phenyl]-1-methyl-3-n-propyl-1,6-dihydro-7H-pyrazolo[4,3-d]pyrimidin-7-thionc (In formula II, wherein R₁ is n-propyl, R₂ is methyl, R₃ is ethyl, and R₄ and R₅ are methyl) (Compound (2))

Step 1. Synthesis of 4-(2-ethoxybenzoylainido)-1-methyl-3-n-propyl-pyrazolyl-5-formamide

The title compound was synthesized by taking 1-methyl-3-n-propyl-4-amino-pyrazolyl-5-formamide as the starting material, according to the step I in example 1. Yield: 75.1%.

The melting point of the product: m.p. 155.5-56.5°C; MS (FAB), 331(M+1).

Step 2. Synthesis of 5-(2-ethoxyphenyl)-1-methyl-3-n-propyl-1,6-dihydro-7H-pyrazolo[4,3-d]pyrimidin-7-one

The title compound was synthesized by taking 4-(2-ethoxybenzoylamido)-1-methyl-3-n-propylpyrazol-5-formamide as the starting material, according to the step 2 in example 1, Yield: 82.6%.

The melting point of the product: m.p. 147-149°C;

Elemental analysis: C₁₇H₂ON₄O₂ calculated: C 65.38%, H 6.41%, N 17.95%, found: C 65.47%, H 6.28%, N 17.74%;

MS(FAB) 313(M+1).

Step 3. Synthesis of 5-(2-ethoxyphenyl)-1-methyl-3-n-propyl-1,6-dihydro-7H-pyrazolo[4,3-d]pyrimidin-7-thione

The title compound was synthesized by taking 5-(2-ethoxyphenyl)-1-methyl-3-n-propyl-1,6-dihydro-7H-pyrazolo[4,3-d]pyrimidin-7-one as the starting material, according to the step 3 in example 1. Yield: 78.7%

The melting point of the product: m.p. 156-158°C;

¹HNMR(DMSO-d₆) (ppm): δ0.94(t,3H), δ1.39 (t,3H), δ1.77(q,2H),δ 2.83(t,2H), δ4.19(q,2H), δ4.44(s,3H), δ7.11(t,1H), δ7.19(d,1H), δ7.53(t,1H), δ7.83(d,1H), δ13.29(s,1H);

MS (FAB) 329(M+1).

Step 4. Synthesis of 5-(5-chlorosulfonyl-2-ethoxyphenyl)-1-methyl-3-n-propyl-1,6-dihydro-7H-pyrazolo[4,3-d]pyrimidin-7-thione

The title compound was synthesized by taking 5-(2-ethoxyphenyl)-1-methyl-3-n-propyl-1,6-dihydro-7H-pyrazolo[4,3-d]pyrimidin-7-thione as the starting material, according to the step 4 in example 1. Yield: 74.9%.

Step 5. Synthesis of 5-[2-ethoxy-5-(cis-3,5-dimethylpiperazine-1-sulfonyl)phenyl]-1-methyl-3-n-propyl-1,6-dihydro-7H-pyrazolo[4,3-d]pyrimidin-7-thione (compound (2))

1.5g of cis-2,6-dimethylpiperazine was dissolved in 10ml of chloroform in a 100-ml three-necked round-bottom flask, to which 3.0g of the product of step (4) was added with cooling in an ice-bath. After the addition, the reactants were continued stirring for another 1 hour. Upon the completion of the reaction, the reaction mixture was washed with aqueous sodium carbonate and water, dried, concentrated, and re-crystallized with ethyl acetate, to give 2.8g of a pale yellow solid.
Yield: 79.0%.

The melting point ofthe product: m.p.196-199°C; MS(FAB)505(M+I);

¹HNMR(DMSO-d₆) (ppm): δ0.92-0.96(m,9H), δ1.36(t,3H), δ1.71-1.79(m,4H), δ2.74-2.78(m,2H), δ2.82(t,2H), δ3.47(m,2H), δ4.24 (q,2H), δ4.45(s,3H), δ7.38(d,1H), δ7.84(dd,1H), δ7.94(d,1H);

The product is C₂₃H₃₂N₆O₃S₂ by elemental analysis.

Example 3. Synthesis of 5-[2-methoxy-5-(cis-3,5-dimethylpiperazin-1-sulfonyl)phenyl]-1-methyl-3-n-propyl-1,6-dihydro-7H-pyrazolo[4,3-d]pyrimidin-7-thione
(In formula II, wherein R₁ is n-propyl, R₂ is methyl, R₃ is methyl, and R₄ and R₅ is methyl) (compound (3))

Step 1. Synthesis of 4-(2-methoxybenzoylamido)-1-methyl-3-n-propyl-pyrazol-5-formamide

The title compound was synthesized by taking 1-methyl-3-n-propyl-4-aminopyrazol-5-formamide and o-methoxybenzoyl chloride as the starting materials, according to the step (1) in example 1. Yield: 74.9%.

The melting point of the product: m.p.125-26°C;

¹HNMR(DMSO-d₆)(ppm): δ0.93(t,J=7.3Hz,3H), δ1.73(q,J=7.3Hz,2H), δ2.77(t,J=7.5Hz,2H), δ3.84(s,3H), δ4.15(s,3H), δ7.07(t,J=7.5Hz,1H), δ7.16 (d,J=8.3Hz,1H), δ7.50(t,J=7.6Hz,1H), δ7.61(d,J=7.5Hz,1H), δ11.95(br,1H);

MS(FAB) 317(M+1).

Step 2. Synthesis of 5-(2-methoxyphenyl)-1-methyl-3-n-propyl-1,6-dihydro-7H-pyrazolo[4,3-d]pyrimidin-7-one

The title compound is synthesized by taking the product of step (1) as the starting material, according to the step 2 in example 1. Yield: 83.8%.

The melting point of the product: m.p.140-141°C; MS(FAB) 299(M+1).

Step 3. Synthesis of 5-(2-methoxyphenyl)-1-methyl-3-n-propyl-1,6-dihydro-7H-pyrazolo[4,3-d]pyrimidin-7-thione

The title compound is synthesized by taking 5-(2-methoxyphenyl)-1-methyl-3-n-propyl-1,6-dihydro-7H-pyrazolo[4,3d]pyrimidin-7-one as the starting material, according to the step 3 in example 1.

The melting point of the product:m.p.160-61°C: MS(FAB) 315(M+1) ;

¹HNMR(DMSO-d₆) (ppm) δ0.93(t,3H), δ1.74(m,2H), δ2.8(t,2H), δ3.87(s,3H), δ4.44(s,3H), δ7.10(t,1H), δ7.20(d,1H), δ7.54(t,1H), δ7.68(d,1H), δ13.38(s,1H).

Step 4. 5-(5-chlorosulfonyl-2-methoxyphenyl)-1-methyl-3-n-propyl-1,6-dihydro-7H-pyrazolo[4,3-d]pyrimidin-7-thione

The title compound is synthesized by taking the product of step (3) as the starting material, according to the step 4 in example 1. Yield: 83.8%.

Step 5. 5-[2-methoxy-5-(cis-3,5-dimethylpiperazin-1-sulfonyl)phenyl]-1-methyl-3-n-propyl-1,6-dihydro-7H-pyrazolo[4,3-d]pyrimidin-7-thione (compound (3))

2.0g of cis-2,6-dimethylpiperazine was dissolved in 5ml of chloroform, to which 3.0 g of product of step 4 was added with cooling in an ice-bath. Upon the completion of the reaction, the reaction mixtures were washed with aqueous Na₂CO₃, dried over Na₂SO₄, and re-crystallized with ethanol, to give 2.8g of a yellow solid. Yield: 78.5%.

The melting point of the product: m.p. 214-215°C;

¹HNMR (DMSO-d₆) (ppm), δ0.91-0.96(m,9H), δ1.72-1.83(m,4H), δ2.79-2.89(m,4H), δ3.53(d,2H), δ3.94(s,3H), δ4.44(s,3H), δ7.39-7.42(m,1H), δ7.87-7.89(m,2H);

MS(FAB) 491(M+1).

The resultant product was confirmed as the desired product.

Example 4. Synthesis of 5-[2-propoxy-5-(cis-3,5-dimethylpiperazin-1-sulfonyl)phenyl]-1-methyl-3-n-propyl-1,6-dihydro-7H-pyrazolo[4,3-d]pyrimidin-7-thione (in formula II, wherein R₁ is n-propyl, R₂ is methyl, R₃ is propyl, and R₄ and R₅ is methyl,) (Compound (4))

Step 1. Synthesis of 4-(2-propoxybenzoylanido)-1-methyl-3-n-propylpyrazol-5-formamide

The title compound is synthesized by taking 1-methyl-3-n-propyl-4-animopyrazol-5-formamide and o-propoxybenzoyl chloride as the starting materials, according to the step I in example 1. Yield: 71.4%.

The melting point of the product: m.p.150-51 °C; MS (FAB) 345(M+1)

Step 2. Synthesis of 5-(2-n-propoxyphenyl)-1-methyl-3-n-propyl-1,6-dihydro-7H-pyrazolo[4,3-d]pyrimidin-7-one

The title compound is synthesized by taking 4-(2-propoxybenzoylamido)-1-methyl-3-n-propylpyrazol-5-formamide as the starting material, according to the step 2 in example 1. Yield: 86.0%.

The melting point of the product: m.p.115-16°C; MS(FAB) 327(M+1).

Step 3. Synthesis of 5-(2-n-propoxyphenyl)-1-methyl-3-n-propyl-1,6-dihydro-7H-pyrazolo[4,3-d]pyrimidin-7-thione

The title compound is synthesized by taking 5-(2-n-propoxyphenyl)-1-methyl-3-n-propyl-1,6-dihydro-7H-pyrazolo[4,3-d]pyrimidin-7-one as the starting material, according to the step 3 in example 1.

The melting point of the product: m.p.111-112°C; MS(FAB) 343(M+1);

¹HNMR (DMSO-d₆) (ppm), δ0.94(1,3H), δ0.999(1,3H), δ1.77(m,4H), δ2.83(t,2H), δ4.09(t,2H), δ4.44(s,3H), δ7.11(t,1H), δ7.20(d,1H), δ7.53(t,1H), δ7.81(t,1H), δ13.26(s,1H).

Step 4. Synthesis of 5-(5-chlorosulfonyl-2-n-propoxyphenyl)-1-methyl-3-n-propyl-1,6-dihydro-7H-pyrazolo[4,3-d]pyrimidin-7-thion

The title compound is synthesized by taking 5-(2-n-propoxyphenyl)-1-methyl-3-n-propyl-1,6-dihydro-7H-pyrazolo[4,3-d]pyrimidin-7-thione as the starting material, according to the step 4 in example 1.

Step 5. Synthesis of 5-[2-propoxy-5-(cis-3,5-dimethylpiperazine-1-sulfonyl)phenyl]-1-methyl-3-n-propyl-1,6-dihydro-7H-pyrazolo[4,3-d]pyrimidin-7-thione (compound (4))

2.0g of cis-2,6-dimethylpiperazine was dissolved in 5ml of chloroform, to which 3.5 g of product of step 4 was added with cooling in an ice-bath. Upon the completion of the reaction, the reaction mixtures were washed with aqueous Na₂CO₃, dried over Na₂SO₄, and re-crystallized with ethanol, to give 3.2g of a yellow solid. Yield: 76.8%.

The melting point of the product: m.p.222-226°C;

¹HNMR(DMSO-d₆)(ppm): δ0.89-0.99(m,12H), δ1.71-1.83(m,6H), δ2.80-2.84(m,4H), δ3.51(d,2H), δ4.15(t,2H), δ4.45(s,3H), δ7.39(d,1H), δ7.85(dd,1H), δ7.95(d,1H);

MS (FAB) 519(M+1).

The resultant product was confirmed as the desired product.

Example 5. Synthesis of 5-[2-ethoxy-5-(cis-3,5-dimethylpiperazine-1-sulfonyl)phenyl]-1-ethyl-3-n-propyl-1,6-dihydro-7H-pyrazolo[4,3-d]pyrimidin-7-thione (in formula II, wherein R₁ is n-propyl, R₂ is ethyl, R₃ is ethyl, and R₄ and R₅ are inethyl) (compound (1))

Step 1. Synthesis of 5-(5-chlorosulfonyl-2-ethoxyphenyl)-1-ethyl-3-n-propyl-1,6-dihydro-7H-pyrazolo[4,3-d] pyrimidin-7-one

The title compound is synthesized by taking the product (5-(2-ethoxyphenyl)-1-ethyl-3-n-propyl-1,6-dihydro-7H-pyrazolo[4,3-d]pyrimidin-7-one) produced in step 2 of example 1 as the starting material, according to the step 4 in example 1. Yield: 80.2%.

Step 2. Synthesis of 5-[2-ethoxy-5-(cis-3,5-dimethylpiperazine-1-sulfonyl)phenyl]-1-ethyl-3-n-propyl-1,6-dihydro-7H-pyrazolo[4,3-d]pyrimidin-7-one

The title compound is synthesized by taking the product of step 1 as the starting material, according to the step 5 in example 1. Yield: 82-3%.

Step 3. Synthesis of 5-[2-ethoxy-5-(cis-3,5-dimethylpiperazine-1-sulfonyl)phenyl]-1-ethyl-3-n-propyl-1,6-dihydro-7H-pyrazolo[4,3-d]pyrimidin-7-thione

4.5g of the product of step 2 dissolved in 30ml of toluene and 1.5g of 2,4-bis(p-metboxyphenyl)-1,3-dithia-2,4-diphosphetane-2,4-disulfide (Lawesson reagent) were added into a 100-ml three-necked round-bottomed flask, and refluxed for 3 hours. The reaction was monitored by thin layer chromatography (developer: chloroform/methanol 100:1). Upon the completion of the reaction, the toluene was removed under reduced pressure. The reaction mixture was cooled to room temperatue, dissolved by the addition of chloroform, dissolved and washed with 2N NaOH, washed 2 times with water. The organic layer was dried over Na₂SO₄, concentrated, and recrystallized with ethanol, to give a yellow solid. Yield: 87.5%.

Example 6. Preparation of 5-[2-ethoxy-5-(cis-3,5-dimethylpiperazin-1 -sulfonyl)phenyl]-1-methyl-3-n-propyl-1,6-dihydro-7H-pyrazolo[4,3-d]pyrimidin-7-thione·citrate

0.25g of citrate monohydrate was dissolved in 5ml of methanol. To this solution, 0.5g of compound (2) obtained in example 2 was added with rapid stirring. Then, the solution was stirred for 1h. The reaction mixture was cooled, crystallized, filtered, and washed, to give 0.6g of a yellow solid. Its melting point is m.p.200-202°C.

Other salts of the compounds of the prevent invention can be made using the similar methods.

Example 7. Test of the pyrazolopyrimidinethione derivatives of the invention on penile erection in rats with testes removed

Methods: Forty healthy adult male wistar rats were used in the study, with the body weight ranging from 230g to 250g. The rats were anesthetized with sodium pentobarbital, and their testes were squeezed into the scrotum. After sterilization, the scrotum was dissected with an operation knife. The testes and epididymis were taken out respectively and then ligated. The wound were sutured and 100,000 unit of penicillin was administrated to each rat for three consecutive days. After breeding for one week, the rats were used for the tests. The animals may be used repeatedly and the interval between any two of the tests was one week. The rats were orally administered with the following compounds: compound (1), compound (2), compound (3), and compound (4), each at the doses of 40mg/kg and 20mg/kg, respectively. Sildenafil at the dose of 50mg/kg was used as the positive control. The control group is (1% sodium carboxymethyl cellulose). In 35min after the administration, the penis of the rats was stimulated with SEN-3201 stimulator (NIHON KOHDEN, Japan), with the following parameters: the stimulating voltage of 10V, the time interval (T1) of 250msec, the deferment time (T2) of 10msec, the duration (T3) of 500µsec, and the stimulation rank (n) of 25. At the same time, the latent period (sec) of the penile erection and the penile swelling time (min) were recorded with a three-channel stop-watch.

The result was shown in table 1. The result indicated that compounds (1), (2), (3), and (4) have evidently shortened the latent period of the penile erection (P<0.001) and lengthened the penile swelling time in the rats with testis removed. Among the compounds, compounds (1) and (2) exhibit more dramatic effects (P<0.001). There was a clear dose-effect relationship. The penile swelling time in the rats administered with compounds (1) and (2) is more evident than that of those administrated with the positive control pharmaceutical sildenafil.

**Table 1. Effect ofthe compounds of the invention on the latent period of penile erection caused by the stimulating electric current and the penile swelling time in rats with testis removed.**

| Compound | Dose (mg/kg) | Animal Number | Latent period (second) | Swelling time (second) |
|---|---|---|---|---|
| Control | | 8 | 53.0±8.7 | 78±16.4 |
| Compound (1) | 20 | 8 | 28.8±1.7*** | 1305.1±588.8*** |
| Compound (1) | 40 | 8 | 29.5±4.2*** | 2943.1±552.9***^{#} |
| Compound (2) | 10 | 8 | 29.5±4.2*** | 1069.8±621.5*** |
| Compound (2) | 20 | 8 | 29.5±4.2*** | 1480.5±469.7*** |
| Compound (2) | 40 | 8 | 30.1±3.9*** | 2675.5±769.2***^{#} |
| Compound (3) | 20 | 8 | 32.8±4.4*** | 551.8±350.8* |
| Compound(3) | 40 | 8 | 30.4±2.6*** | 809.6±247.5** |
| Compound(4) | 20 | 8 | 31.8±3.1*** | 304.5±174.1** |
| Compound (4) | 20 | 8 | 31.9±4.5*** | 829.9±550.2 |
| Sildenafil | 50 | 8 | 28.9±1.7*** | 2272.5±326.2*** |

| | | | | |
|---|---|---|---|---|
| *P<0.05, **P<0.01, ***P<0.001, compared with the control group; ^{#}P<0.05, compared with Sildenafil. | | | | |

Example 8. Test of the duration of penile erection caused with compound (2) in rats with testis removed.

Methods: Forty healthy adult male wistar rats were used in the study, with the body weight ranging from 240g to 270g. The rats were orally administered with compound (2) at the dose of 40mg/kg, 20mg/kg, and 10mg/kg, respectively. The positive control Sildenafil was administrated at the dose of 50mg/kg. And the control group was administrated with 1% sodium carboxymethyl cellulose. At 1h, 3h, 6h, and 24h after the administration, the penises of the rats were stimulated by SEN-3201 stimulator (NIHON KOHDEN, Japan), with the following parameters: the stimulating voltage of 10V, the time interval (T1) of 250msec, the deferment time (T2) of 10msec, the duration (T3) of 500µsec, and the stimulation rank (n) of 25. The latent period of penile erection (sec) and the penile swelling time (min) were recorded with three-channel stop watches at the same time.

The result was shown in table 2. The result indicated that compound (2) with the doses of 40mg/kg and 20mg/kg can make the penile swelling of the rats with testis removed last for up to 24 hours. There was a clear dose-effect relationship. The effect was apparently better than that of Sildenafil used as the positive control. For the rats administrated with Sildenafil, the penile swelling time was about I hour, and the penile swelling time was shorter than that of the control group at 3 hr after the administration.

**Table 2. Effect of compound (2) on the duration of penile erection in rats with testis removed**

| Group | Dose mg/kg | Penile swelling time (seconds) | | | | |
|---|---|---|---|---|---|---|
| | | Value before administration | At 1h after administration | At 3h after administration | At 6h after administration | At 24h after administration |
| Control group | | 77.6±28.2 | 78.0±16.4 | 63.6±20.6 | 78.1±49.4 | 66.0±16.8 |
| compound(2) | 10 | 67.7±12.3 | 679.6±513.2*** | 544.7±166.9*** | 268.7±146.2*** | 98.6±57.9 |
| Compound (2) | 20 | 82.4±26.6 | 1514.8±258.9*** | 906.0±406.7*** | 275.8±154.2*** | 201.0±85.7** |
| Compound(2) | 40 | 84.0±18.4 | 2187.1±547.8*** | 783.0±329.2*** | 678.7±221.8*** | 194.0±65.1** |
| Sildenafil | 50 | 62.8±7.7 | 1067.0±204.5*** | 44.0±11.3^{###} | 38.8±5.6^{###} | 61.0±8.7^{##} |

| | | | | | | |
|---|---|---|---|---|---|---|
| *P<0.05, **P<0.01, ***P<0.001, compared with the control group; ^{###}P<0.05, Sildenafil, compared with compound (2). | | | | | | |

Example 10. Effect of the pyrazolopyrimidinethione derivatives of the invention on the sexual function of testis-removed mice

Methods: Forty healthy KM male mice with the body weight ranging from 28-32g and forty healthy KM female mice with body weight ranging from 24-28g were used. Each male mouse was anesthetized with sodium pentobarbital, and the testes were squeezed into the scrotum. After sterilization, the scrotum was dissected with an operating knife. The testes and epididymis were respectively taken out, and then ligated. The wound were sutured and each of the mice was administrated with 100, 000 units of penicillin for three consecutive days. After being bred for one week, the mice were used for the tests. The animals may be employed repeatedly and the interval between any two tests was 72 hours. To the mice, the following compounds were orally administered: compound (I), compound (2), compound (3), and compound (4), each at the dose of 80mg/kg and 40mg/kg, respectively. The positive control agent was Sildenafil, at the dose of 80mg/kg. The control group was given with 1% of sodium carboxymethyl cellulose. At 30min after the administration, the male mice who had taken the above agents were put into the cages of female mice, with the male-to-female rare of 1: 1. There were three males and three females in each cage. The times of back climbing on the female mice performed by the male mice, and the percentage of occurrence were observed within 2 hours after the administration.

The results were shown in table 3. The results indicated that the times of back climbing on female mice by male mice were 11, 20, and 16 respectively within 2 hours after the male mice were orally administrated compound (2) at the dose of 40mg/kg and 80mg/kg, and the control pharmaceutical Sildenafil 80mg/kg. The incidences of the back climbing were 60%, 80%, and 70%. This exhibited a significant difference (P<0.01), in comparison with the control group with one back climbing and the incidence being 10 %. The result of compound (2) was the same as that of the positive control pharmaceutical Sildenafil.

**Table 3. Effect of the pyrazolopyrimidinethione derivatives of the invention on the sexual function of mice with testis removed.**

| Compound | Dose (mg/kg) | Animal number | Number of the animals who performed back climbing | Times of back climbing | Incidence (%) |
|---|---|---|---|---|---|
| Control group | | 10 | 1 | 1 | 10 |
| Compound (1) | 80 | 10 | 4 | 7* | 40 |
| Compound (1) | 40 | 10 | 3 | 4 | 30 |
| Compound (2) | 80 | 10 | 8** | 20** | 80 |
| Compound (2) | 40 | 10 | 6* | 11* | 60 |
| Compound (3) | 80 | 10 | 3 | 4 | 30 |
| Compound (3) | 40 | 10 | 2 | 2 | 20 |
| Compound (4) | 80 | 10 | 3 | 5 | 30 |
| Compound (4) | 40 | 10 | 2 | 3 | 20 |
| Sildenafil | 80 | 10 | 6* | 16** | 70 |

| | | | | | |
|---|---|---|---|---|---|
| *P<0.05, **P<0.01, compared with control group. | | | | | |

Example 11, The Effect of the pyrazolopyrimidinethione derivatives of the present invention on the blood pressure, the heart rate, and the respiration of the anesthetized dogs.

Methods: eight healthy male dogs with the body weight ranging from 12-15kg were used. The dogs were anesthetized with 30mg/kg of sodium pentobarbital. The femoral artery was dissected and inserted with a blood pressure transducer to record the blood pressure. The limbs were pierced with electrodes for recording the electrocardiogram. The respiration belt was wrapped around their chest and the respiration rate was recorded. The apparatus used was RM-6000 Polygraph system (NIHON KOHDEN, Japan). The changes in the arterial blood pressure, the heart rate, and the respiration were observed after the test compound was introduced into the stomach of the anesthetized dogs via 14# catheter.

The result showed that the arterial blood pressure (systolic pressure and diastolic pressure), the heart rate, and the respiration (respiratory frequency and respiratory depth) of the test dogs did not experience an evident change in 3 hours after the oral administration of compound (1), compound (2), compound (3), and compound (4) at the dose of 40mg/kg. This suggested that pyrazolopyrimidinethione derivates of the invention have no significant effect on the blood pressure, the heart rate and the respiratory of a dog. However, the positive control pharmaceutical Sildenafil (40mg/kg) has an apparent effect of decreasing the blood pressure, mainly decreasing the diastolic pressure of the animal.

Example 12. Effect of compound (2) of the pyrazolopyrimidinethione derivates of the invention on the blood pressure and the heart rate of the anesthetized rats

Forty male wistar rats with the body weight ranging from 220-240g were used in the study. Pharmaceuticals: compound (2) and Sildenafil, which were ground and then suspended with 1% of sodium carboxymethyl cellulose. Apparatus: RM-6000 eight-graph system (NIHON KOHDEN, Japan). The doses for administration and the division of the groups: solvent control group (abbreviated as the control group), 20mg/kg of compound (2), 40mg/kg of compound (2), 80mg/kg of compound (2), and 50mg/kg of Sildenafil. The administration route is through dodecadactylon. Methods: the male wistar rats were taken out and anesthetized with 35mg/kg of sodium pentobarbital by injecting into the abdominal cavity, on the basis of their body weight. The rats were fixed on the operation table and were performed with the dissection operation of right carotid artery. The catheter with an external diameter of 1mm was inserted into the right carotid artery. The blood pressure was measured and the electrodes positioned on the skin of the four limbs were used to record the electrocardiogram limb lead II. The catheter with an external diameter of 2mm was inserted into the dodecadactylon via incision of the epigastric region, for the administration of the pharmaceuticals. RM-6000 eight-graph system (NIHON KOHDEN, Japan) was employed to record. Observation time: the value before administration, the values at 15, 30, 60, 120 and 180 min after the administration.

1. The effect of compound (2) on the heart rate of the anesthetized rats was shown in table 4. The results indicated that compound (2) administrated through dodecadactylon did not exhibit an evident effect on the heart rate of the anesthetized rats, at the doses of 20mg/kg, 40mg/kg, and 80mg/kg, respectively. Sildenafil with a dose of 50mg/kg has the effect of increasing the heart rate of the rats at 15 and 30 minutes after the administration.

**Table 4. Effect of compound (2) on the heart rate of the anesthetized rats**

| Group | Dose (mg/kg) | Animal number | Heart rate (times/min) | | | | |
|---|---|---|---|---|---|---|---|
| | | | Before administration | At 15 min after administration | At 30 min after administration | At 60 min after administration | At 120 min after administration |
| Control | | 8 | 421±32.6 | 423±29.7 | 424±29.6 | 425±22.9 | 424±22.6 |
| Compound (2) | 20 | 8 | 445±25.1 | 440±29.7 | 440±29.6 | 436±22.9 | 428±22.6 |
| Compound (2) | 40 | 8 | 429±42.8 | 422±29.7 | 427±29.6 | 425±22.9 | 424±22.6 |
| Compound (2) | 80 | 8 | 439±22.6 | 439±29.7 | 442±29.6 | 436±22.8 | 438±22.6 |
| Sildenafil | 50 | 8 | 429±26.1 | 445±29.7 | 444±29.6 | 437±22.8 | 439±22.6 |

**Table 5. Effect of compound (2) on the systolic pressure of the anesthetized rats.**

| Group | Dose (mg/kg) | Animal number | Systolic pressure (mmHg) | | | | |
|---|---|---|---|---|---|---|---|
| | | | Value before administration | At 15 min after administration | At 30 min after administration | At 60 min after administration | At 120 min after administration |
| Control | | 8 | 130±13.7 | 132±29.7 | 131±29.6 | 130±22.9 | 134±22.6 |
| compound(2) | 20 | 8 | 134±7.2 | 132±29.5 | 134±29.6 | 128±22.9 | 127±22.6 |
| compound(2) | 40 | 8 | 129±13.6 | 130±29.7 | 134±29.6 | 122±22.9 | 133±22.6 |
| compound(2) | 80 | 8 | 126±12.1 | 121±29.7 | 123±29.6 | 121±22.9 | 119±22.6 |
| Sildenafil | 50 | 8 | 120±10.6 | 90±29.7*** | 90±29.6*** | 88±22.9*** | 93±22.6** |

| | | | | | | | |
|---|---|---|---|---|---|---|---|
| ** P<0.01, *** P<0.001, compared with control group and value before administration. | | | | | | | |

**Table 6. Effect of compound (2) on the diastolic pressure of the anesthetized rats**

| Group | Dose (Mg/kg) | Animal number | diastolic pressure (mmHg) | | | | |
|---|---|---|---|---|---|---|---|
| | | | Value before administration | At 15 min after administration | At 30 min after administration | At 60 min after administration | At 120 min after administration |
| Control | | 8 | 109±14.1 | 110±29.7 | 109±29.6 | 108±22.9 | 112±22.6 |
| Compound(2) | 20 | 8 | 109±14.1 | 108±29.7 | 104±29.6 | 101±22.9 | 102±22.6 |
| Compound(2) | 40 | 8 | 109±14.4 | 109±29.7 | 109±29.6 | 109±22.9 | 112±22.6 |
| Compound (2) | 80 | 8 | 102±12.0 | 102±29.7 | 97±29.6 | 96±22.9 | 94±22.6 |
| Sildenafil | 50 | 8 | 98±16.0 | 68±29.7*** | 70±29.6*** | 68±22.9*** | 71±22.6** |

| | | | | | | | |
|---|---|---|---|---|---|---|---|
| **P<0.01, ***P<0.001, compared with control group and value before administration. | | | | | | | |

2. The effect of compound (2) on the blood pressure of the anesthetized rats was shown in tables 5 and 6. The results indicated that compound (2) administrated through dodecadactylon did not exhibit an evident effect on the systolic pressure and diastolic pressure of the rats at the doses of 20mg/kg, 40mg/kg, and 80mg/kg. The blood pressure began to decrease at 15 min after the administration of Sildenafil at the dose of 50mg/kg, and the blood pressure for the majority of the animals did not restore to the level before administration, with the blood pressure reduction of some individual animals lasting up to 4 or 5 hours.

Example 13. Test of the preliminary acute toxicity of the pyrazolopyrimidinethione derivatives of the invention

Experimental methods: the toxicity test was conducted by oral and intragastric administration of the pyrazolopyrimidinethione derivatives of the invention using the Bliss method. The 50% lethal dose LD₅₀ that the pharmaceuticals kill the rats was: compound (1), LD₅₀>3000mg/kg, 95% confidence limit >2500-3000g/kg; compound (2), LD₅₀>3000mg/kg, 95% confidence limit >3000g/kg mg/kg; compound (3), LD₅₀ 2500mg/kg, 95% confidence limit 2500-3000mg/kg; compound (4), LD₅₀>3000mg/kg, 95% confidence limit >3000mg/kg. For Sildenafil, the LD₅₀ was 635mg/kg with the 95% confidence limit of 50-672mg/kg, as reported in *Chinese Journal of Clinical Pharmacology and Therapeutics* (1999, 4(3):237-240).

The results showed that LD₅₀ for compound (2) was greater than 3000mg/kg, indicating that the pyrazolopyrimidinethione derivatives of the invention has very low toxicity, which is apparently lower than thatofthe control pharmaceutical Sildenafil.

### INDUSTRIAL APPLICATION

The compounds of the invention are potent inhibitors of cyclic guanosine 3', 5'-monophosphate phosphodiesterases (cGMP PDEs). The pharmaceuticals, which utilize the invented compounds for preventing and/or treating impotence and frigidity, have the advantages of high selectivity for phosphodiesterase V, long-term efficacy, and small cardiovascular side effects. The pharmaceuticals will not decrease the blood pressure and increase the heart rate. Thereby the pharmaceudcals will possess a good future in markets.

## Claims

1. Pyrazolopyrimidinethione derivatives having the structure of formula I : 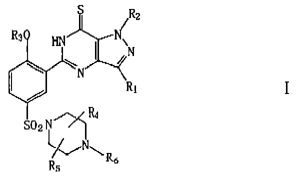 Wherein: R₁, R₂, and R₃ are same or different, and independently are alkyl having 1-6 carbon atoms, alkyl having 1-6 carbon atoms in which at least one hydrogen atom is substituted by alkoxy having 1-6 carbon atoms or cycloalkyloxy having 3-6 carbon atoms, alkenyl having 2-6 carbon atoms, or aryl having 6-10 carbon atoms;
R₄ is alkyl having 1-6 carbon atoms, alkenyl having 2-6 carbon atoms, alkoxy having 1-6 carbon atoms, cycloalkyloxy having 3-6 carbon atoms, or aryl having 6-10 carbon atoms;
R₅ is hydrogen, alkyl having 1-6 carbon atoms, alkenyl having 2-6 carbon atoms, alkoxy having 1-6 carbon atoms, cycloalkyloxy having 3-6 carbon atoms, or aryl having 6-10 carbon atoms;
R₆ is hydrogen, alkyl having 1-6 carbon atoms, alkenyl having 3-6 carbon atoms, cycloalkyl having 3-8 carbon atoms, or alkyloyl having 1-6 carbon atoms.

2. The pyrazolopyrimidinethione derivatives according to claim 1, **characterized in that**: said derivatives have the structure of formula II, 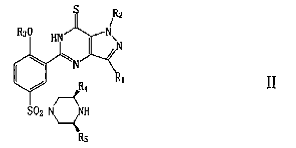 Wherein, R₁, R₂, R₃, R₄, and R₅ dependently are alkyl having 1-6 carbon atoms.

3. The pyrazolopyrimidinethione derivatives according to claim 1, wherein said pyrazolopyrimidincthione derivatives are:
5-[2-methoxy-5-(cis-3,5-dimethylpipcrazin-1-sulfonyl)phenyl]-1-methyl-3-n-propyl-1,6-dihydro -7H -pyrazolo[4,3-d]pyrimidin-7-thione;
5-[2-ethoxy-5-(cis-3,5-dimethylpiperassin-1-sulfonyl)phenyl]-1-methyl-3-n-propyt-1,6-dihydro-7H -pyrazolo[4,3-d]pyrimidin-7-thione;
5-[2-propoxy-5-(cis-3,5-dimethylpiperazin-1-sulfonyl)phenyl]-1-methyl-3-n-propyl-1,6-dihydro -7H-pyrazolo[4,3-d]pyrimidin-7-thione;
5-[2-methoxy-5-(cis-3,5-dimethylpiperazin-1-sulfonyl)phenyl]-1-ethyl-3-n-propyl-1,6-dihydro-7H -pyrazolo[4,3-d]pyrimidin-7-thione;
5-[2-ethoxy-5-(cis-3,5-dimethylpiperazin-1-sulfonyl)phenyl]-1-ethyl-3-n-propyl-1,6-dihydro-7H -pyrazolo[4,3-d]pyrimidin-7-thione; or
5-[2-propoxy-5-(cis-3,5-dimethylpiperazin-1-sulfonyl)phenyl]-1-ethyl-3-n-propyl-1,6-dihydro-7 H -pyrazolo[4,3-d]pyrimidin-7-thione.

4. The salts of pyrazolopyrimidinethione derivatives according to any one of claims 1-3, **characterized in that**: said salts are salts of organic acids or inorganic acids.

5. The salts according to claim 4, **characterized in that**: said salts of organic acids are citrate, fumarate, oxalate, inalate, lactate, camphorsulfonate, p-toluenesulfonate, or methanesulfonate; said salts of inorganic acids are salts of haloid acid, sulfate, phosphate, or nitrate.

6. The solvates of the compounds according to any one of claims 1-5, **characterized in that**: the solvents are water, ethanol, or methanol.

7. A method for preparing the pyrazolopyrimidinethione derivatives of claim 1, comprising reacting the compound of formula III with the compound of formula IV to give said pyrazolopyrimidinethione derivatives; 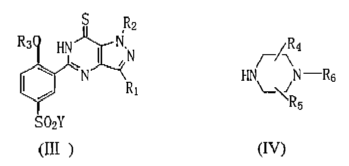 Wherein: in the compounds of formulas III and IV, R₁, R₂, and R₃ are same or different, and independently are alkyl having 1-6 carbon atoms, alkyl having 1-6 carbon atoms in which at least one hydrogen atom is substituted by alkoxy having 1-6 carbon atoms or cycloalkyloxy having 3-6 carbon atoms, alkenyl having 2-6 carbon atoms, or aryl having 6-10 carbon atoms;
R₄ is alkyl having 1-6 carbon atoms, alkenyl having 2-6 carbon atoms, alkoxy having 1-6 carbon atoms, cycloalkyloxy having 3-6 carbon atoms, or aryl having 6-10 carbon atoms;
R₅ is hydrogen, alkyl having 1-6 carbon atoms, alkenyl having 2-6 carbon atoms, aryl having 6-10 carbon atoms, or alkyloyl having 1-6 carbon atoms;
R₆ is hydrogen, alkyl having 1-6 carbon atoms, alkenyl having 3-6 carbon atoms, cycloalkyl having 3-8 carbon atoms, or alkyloyl having 1-6 carbon atoms; and
Y is Cl, F, Br, or I.

8. The method according to claim 7, **characterized in that**: the solvents used in the reaction are chloroform, tetrahydrofuran, dioxane, ethanol, 1,2-dimethoxyethane, xylene, toluene, dimethyl sulfoxide, or triethylamine.

9. A method for preparing the pyrazolopyrimidinethione derivates of claim 1, comprising firstly reacting the compound of formula V with the compound of formula IV to give the compound of formula VI, and then sulfurizing said compound of formula VI to give said pyrazolopyrimidinethione derivatives; 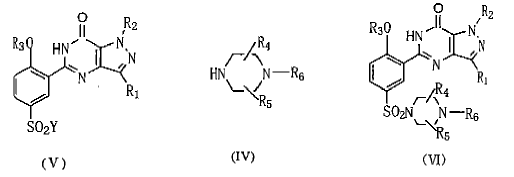 Wherein: in the compounds of formula IV, V, and VI, R₁, R₂, and R₃ are same or different, and independently are alkyl having 1-6 carbon atoms, alkyl having 1-6 carbon atoms in which at least one hydrogen atom is substituted by alkoxy having 1-6 carbon atoms or cycloalkyloxy having 3-6 carbon atoms, alkenyl having 2-6 carbon atoms, or aryl having 6-10 carbon atoms;
R₄ is alkyl having 1-6 carbon atoms, alkenyl having 2-6 carbon atoms, alkoxy having 1-6 carbon atoms, cycloalkyloxy having 3-6 carbon atoms, or aryl having 6-10 carbon atoms;
R₅ is hydrogen, alkyl having 1-6 carbon atoms, alkenyl having 2-6 carbon atoms, aryl having 6-10 carbon atoms, or alkyloyl having 1-6 carbon atoms;
R₆ is hydrogen, alkyl having 1-6 carbon atoms, alkenyl having 3-6 carbon atoms, cycloalkyl having 3-8 carbon atoms, or alkyloyl having 1-6 carbon atoms; and
Y is Cl, F, Br, or I.

10. The method according to claim 9, **characterized in that**: the solvent for sulfurization reaction is tetrahydrofuran, dioxane, 1,2-dimethoxyethane, ethanol, xylene, toluene, dimethyl sulfoxide, or triethylamine.

11. The method according to claim 10, **characterized in that**: the sulfurating reagent for said sulfurization is phosphorus pentasulfide or 2,4-Bis(p-methoxyphenyl)-1,3-dithia-2,4-diphosphetane-2,4-disulfide, and derivatives thereof, and the temperature is-20-200°C.

12. A method for preparing the salts of pyrazolopyrimidinethione derivatives of claim 4, comprising reacting said pyrazolopyrimidinethione derivatives of claim 1 wth the pharmaceutically acceptable acids to give said salts.

13. A pharmaceutical comprising the pyrazolopyrimidinethione derivatives of claim 1, or 2, or 3 as the active ingredient, for preventing and/or treating impotence.

14. A pharmaceutical comprising the pyrazolopyrimidinethione derivatives of claim 1, or 2, or 3 as the active ingredient, for preventing and/or treating frigidity.

15. A pharmaceutical comprising salts of the pyrazolopyrimidinethione derivatives of claim 4 or 5 as the active ingredient, for preventing and/or treating impotence.

16. A pharmaceutical comprising salts of the pyrazolopyrimidinethione derivatives of claim 4 or 5 as the active ingredient, for preventing and/or treating frigidity.

17. A pharmaceutical comprising solvates of the pyrazolopyrimidinethione derivatives of claim 6 as the active ingredient, for preventing and/or treating impotence.

18. A pharmaceutical comprising solvates of the pyrazolopyrimidinethione derivatives of claim 6 as the active ingredient, for preventing and/or treating frigidity.

19. A pharmaceutical comprising the pyrazolopyrimidinethione derivatives of claim 1, or 2, or 3, or salts or solvates thereof, as the active ingredient for preventing and/or treating impotence and frigidity.

20. The pharmaceutical according to claim 19, **characterized in that**: said pharmaceutical further comprises a pharmaceutically acceptable diluent or carrier.
